(19) Europäisches Patentamt
European Patent Office
Office européen des brevets

(11) Publication number: 0 431 953 A2

# (12) EUROPEAN PATENT APPLICATION

(21) Application number: **90313296.7**

(22) Date of filing: **07.12.90**

(51) Int. Cl.5: **C07D 487/04**, A61K 31/505, //(C07D487/04,239:00,239:00)

A request for correction in pages 17, 30 and 32 has been filed pursuant to Rule 88 EPC. A decision on the request will be taken during the proceedings before the Examining Division (Guidelines for Examination in the EPO, A-V, 2.2).

(30) Priority: **08.12.89 JP 320174/89**

(43) Date of publication of application: **12.06.91 Bulletin 91/24**

(84) Designated Contracting States: **AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Akimoto, Hiroshi**
**4-25, Morikitamachi 6-chome,**
**Higashinada-ku**
**Kobe, Hyogo 658(JP)**
Inventor: **Hitaka, Takenori**
**22-302, 1 Nakayamasatsukidai 6-chome**
**Takarazuka, Hyogo 665(JP)**

(74) Representative: **Laredo, Jack Joseph et al**
**Elkington and Fife Beacon House 113**
**Kingsway**
**London, WC2B 6PP(GB)**

## (54) Pyrrolopyrimidine derivatives, their production and use.

(57) Compounds of the formula:

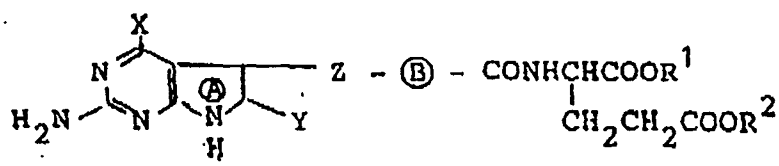

wherein the ring Ⓐ is a pyrrole ring which may be hydrogenated; X is an amino, hydroxyl or mercapto group; Y is a hydrogen or hydroxyl group; $-COOR^1$ and $-COOR^2$ independently represents a carboxyl group which may be esterified; - Ⓑ - is a cycloalkylene or cycloalkenylene group which maybe substituted or a phenylene group which is substituted; Z is a divalent $C_{2-4}$ aliphatic group of straight chain which may be substituted or its salt. Their production is characterized by reacting the compounds of the formula,

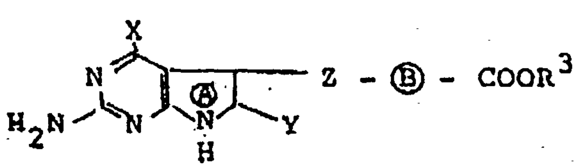

wherein $-COOR^3$ is a carboxyl group which may be esterified; Ⓐ , X, Y, Z and - Ⓑ - are the same as defined above or their reactive derivatives in the carboxyl group with the compounds of the formula,

EP 0 431 953 A2

Xerox Copy Centre

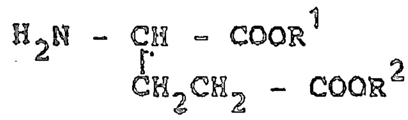

wherein -COOR[1] and -COOR[2] are the same as defined above.

The product compounds and their salts have inhibitory activity against enzymes which utilize folic acid or its related compound as a substrate, and are employable for treating tumors.

# PYRROLOPYRIMIDINE DERIVATIVES, THEIR PRODUCTION AND USE

This invention relates to novel pyrrolo[2,3-d] pyrimidine derivatives which are useful as an anti-tumor agent and to a production process thereof.

Folic acid and its related compounds, as a transferring agent of C1 units derived from formic acid or formaldehyde in living body, play a role as a coenzyme in a great variety of enzymic reactions, such as the nucleic acid biosynthesis, amino acid-peptide metabolism and methane production systems. In the nucleic acid biosynthesis system, particularly, such compounds are essential for the C1 unit metabolism-transfer reactions in the purine synthesis and thymidine synthesis pathways. In order for folic acid to demonstrate its biological activities, normally, it is required to undergo two steps of reduction to be transformed to its active coenzyme forms. As a drug substance which binds strongly to the enzyme (dihydrofolate reductase) governing the second reduction step to thereby inhibit the reduction of dihydrofolate to tetrahydrofolate, there are known amethopterine (methotrexate: MTX) and its analogous compounds. These drug substances, that act to exert damage to DNA synthesis, eventually bringing about cell death, have been developed as anti-tumor agent and occupy a clinically important position. Furthermore, reports have been made of the folic acid antagonists that work by a mechanism being different from the inhibition of dihydrofolate reductase, namely a tetrahydroaminopterine-type anti-tumor agent
(5,10-dideaza-5,6,7,8-tetrahydroaminopterine: DDATHF)
[Journal of Medicinal Chemistry, 28, 914 (1985)] which can act mainly through a mechanism to inhibit glycineamide. ribonucleotide•transformylase being involved in the initial stage of purine biosynthetic pathway or a quinazoline-type anti-tumor agent (2-desamino-2-methyl--10-propargyl-5,8-dideazafolate: DMPDDF) [British Journal of Cancer, 58, 241 (1988)] which can work principally through a mechanism to inhibit thymidylate synthetase participating in the conversion of 2-deoxyuridylic acid to thymidylic acid, and others. Besides those folic acid antagonists having a basic skeleton of the condensed ring from two six-membered rings, on the other hand, it was also reported that the compounds composed of the pyrrolo[2,3-d]pyrimidine skeleton, a condensed ring from a six-membered ring and a five-membered ring, and the glutamic acid moiety crosslinked through a group containing a phenylene group exhibit anti-tumor activity, as well. Referring particularly to the above-mentioned pyrrolo[2,3-d]pyrimidine derivatives, however, the crosslinking portion is limited to groups containing a non-substituted phenylene group alone [The Japanese Unexamined Patent Publication No. 72235/1989].

Currently, it has been especially expected of cancer therapy to develop and create drug substances which possess a novel mechanism of action, exhibit highly selective toxicity against cancer cells and are capable of achieving excellently improved therapeutic effect. MTX, an anti-tumor agent that can work basically through a mechanism to serve the antagnonism against folic acid, is presently put in wide, clinical use but because of its relatively strong toxicity and inadequate efficacy against solid cancers, has failed to attain adequately satisfactory therapeutic effect, while at the same time, the acquired resistance of tumor cells to this kind of drug substances is presenting a great problem.

In view of the above situations, the present inventors conducted repeatedly intensive research and as a result, found that the compounds composed of a pyrrolo[2,3-d]pyrimidine skeleton and a glutamic acid moiety being bound through a crosslinking containing a cycloalkylene or cycloalkenylene group or a phenylene group having a substituent exhibit highly selective toxicity toward a variety of tumor cells and also anti-tumor activity against MTX-resistant cell lines, which has led to completion of this invention.

Namely, this invention is concerned with:

(1) A compound represented by the formula:

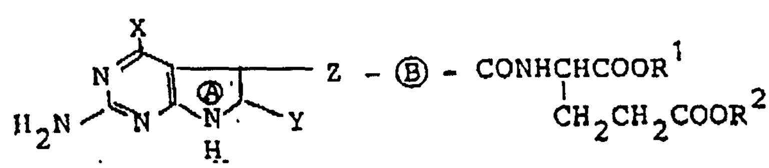 (I)

wherein the ring Ⓐ is a pyrrole ring which may be hydrogenated; X is an amino, hydroxyl or mercapto group; Y is a hydrogen atom or a hydroxyl group; $-COOR^1$ and $-COOR^2$ each is the same as or different from the other and represents a carboxyl group which may be esterified; - Ⓑ - is a cycloalkylene group which may be substituted, a cycloalkenylene group which may substituted or a phenylene group which may be substituted; Z is a divalent $C_{2-4}$ aliphatic group of straight chain which may be substituted, or its

salt:

(2) A process for producing a compound of the item (1), which comprises reacting a compound of the formula,

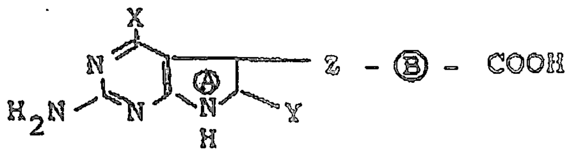

(II)

wherein the rings Ⓐ is a pyrrole ring which may be substituted; X is an amino, hydroxyl or mercapto group; Y is a hydrogen atom or a hydroxyl group; - Ⓑ - is a cycloalkylene group which may be substitueted, a cycloalkenylene group which may be substituted or a phenylene group which is substituted; Z is a divalent $C_{2-4}$ aliphatic group of straight chain which may be substituted, or its reactive derivative in respect to the carboxyl group with a compound of the formula:

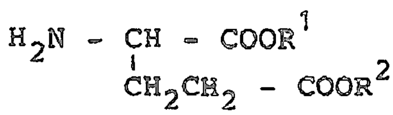

(III)

wherein $-COOR^1$ and $-COOR^2$ each is the same as or different from the other and represents a carboxyl group which may be esterified:

(3) An anti-tumor agent comprising a compound of the item (1) or its pharmaceutically acceptable salt and a pharmaceutically acceptable diluent or carrier:

(4) A compound as represented by the general formula:

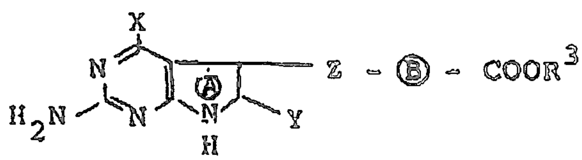

(IV)

wherein the ring Ⓐ is a pyrrole ring which may be hydrogenated; X is an amino, hydroxyl or mercapto group; Y is a hydrogen atom or a hydroxyl group; - Ⓑ - is a cycloalkylene group which may be substituted, a cycloalkenylene group which maybe substituted or a phenylene group which is substituted; $COOR^3$ is a carboxyl which may be substituted; Z is a divalent $C_{2-4}$ aliphatic group of straight chain which may be substituted] or its salt:

(5) A process of producing a compound of the item (4), which comprises (a) reacting a compound represented by the formula:

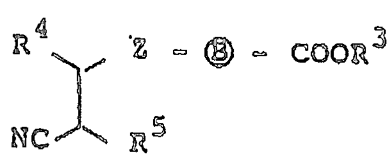

wherein $R^3$ is a carboxyl group which may be esterified; $R^4$ is a carboxyl group which may be esterified; $R^4$ is a cyano group or a carboxyl group which may be esterified; - Ⓑ - is a cycloalkylene group which may be substituted, a cycloalkenylene group which may be substituted or phenylene group which is substituted; Z is a divalent $C_{2-4}$ aliphatic group of straight chain which may be substituted, or a salt thereof with guanidine, or (b) allowing a compound represented by the formula:

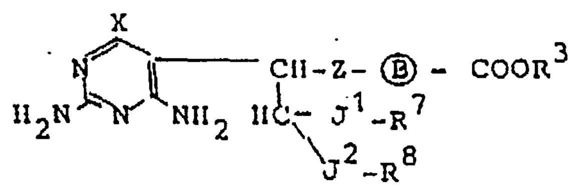

wherein $R^3$ , - Ⓑ - and Z are the same as defined above; X is an amino, hydroxyl or mercapto group; $R^7$ and $R^8$ each is the same or different from the other and represents a hydrocarbon group; $J^1$ and $J^2$ each is the same or different from the other and represents oxygen or sulfur atom, or a salt thereof to undergo a ringclosure reaction and if necessary, followed by a deesterification reaction or/and a reduction reaction in an optional order: and

(6) A method for treating or preventing tumors in mammals which comprises administrating an effective amount of a compound of the item (1) or its pharmaceutically acceptable salt together with a pharmaceutically acceptable carrier or diluent to a subject suffering from a tumor.

In cases where in the above formulae, X is a hydroxyl or mercapto group and Y is a hydroxyl group, the compounds (I), (II) and (III) can exist as an equilibrium mixture with their tautomeric isomers. Illustrated below are the partial structural formulae capable of undergoing tautomerism, with the equilibria among them being shown, as well.

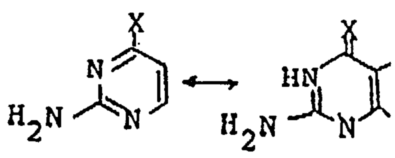

(X=OH,SH X'=O,S)

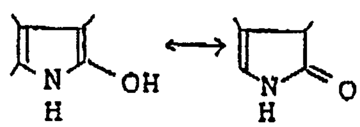

Throughout this specification, for the purpose of convenience of expression, the hydroxyl and mercapto forms are to be described, with the corresponding designations being employed, and in either case, their tautomers or the oxo and thioxo forms are understood to be included therein.

In the compounds (I) of the present invention, the presence of a plurality of asymmetric centers is possible, and except that the absolute configuration of the asymmetric carbon in the side chain derived from the glutamic acid moiety is S(L), the absolute configurations of other centers of asymmetry may be either of S, R and a mixture of S and R. In such a case, a plural number of diastereoisomers exist and can be easily separated into the separate isomers by conventional separation and purification means, if necessary. All the above-mentioned diastereoisomers that are separable by such procedures are understood to be included into the scope of this invention.

Referring to the above formulae, examples of the pyrrole ring as represented by the ring Ⓐ which may be hydrogenated include pyrrole and pyrroline rings, preferably, pyrrole rings.

In the above formula, Z represents a divalent $C_2$ to $C_4$ aliphatic hydrocarbon group of straight chain (more exactly, a group composed of two to four carbon atoms bonded to make a straight chain and zero to eight hydrogen atoms), and as the said divalent group, there may be employed, for example, $C_{2-4}$ alkylenes, such as ethylene, trimethylene and tetramethylene; $C_{2-4}$ alkenylenes, such as vinylene, propenylene and 1- or 2-butenylene; and $C_{2-4}$ alkynylenes, such as ethynylene, 1- or 2-propynylene and 1 - or 2-butynylen. The divalent groups as represented by Z may possess one to two substituents, being same or different, whereby examples of such substituents include alkyl groups having a number of carbon atoms of 1 to 3 (e.g., methyl, ethyl, propyl and isopropyl groups), alkenyl groups having a number Of carbon atoms of 2 to 3 (e.g., vinyl, 1-methylvinyl, 1-porpenyl, allyl and allenyl groups), alkynyl groups having a number of carbon atoms of 2 to 3 (e.g., ethynyl, 1-propynyl and propargyl groups) or cyclopopyl groups as well as fluorine, hydroxyl, oxo, methoxy, dimethylamino, diethylamino, trifluoromethyl, formyl, hydroxymethyl, 2-hydroxyethyl, methoxymethyl, 2-ethoxyethyl, etc preferably methyl, ethyl, propyl, allyl, propargyl, formyl, hydroxymethyl or methoxymethyl.

As the carboxyl group represented by $-COOR^1$, $-COOR^2$ and $-COOR^3$ which may be esterified, there are mentioned, for example, carboxyl groups being esterified with lower alkyl groups having a number of carbon atoms of 1 to 5, benzyl groups which may be substituted or phenyl groups which may be substituted and others preferably $C_{1-5}$ alkyl or benzyl groups. As the said lower alkyl groups, there may be

mentioned, for example, methyl, ethyl, propyl, isopropyl, n-butyl, iso-butyl, sec-butyl, tert-butyl, n-pentyl, iso-pentyl, sec-pentyl, neo-pentyl and tert-pentyl, and the said benzyls which may have substituents include, for example, benzyl, nitrobenzyl and methoxybenzyl, while examples of the said phenyls which may have substituents include phenyl, nitrophenyl and methoxyphenyl.

Preferred examples of the carboxyl group represented by $-COOR^1$, $-COOR^2$ and $-COOR^3$ are carboxyl which may be esterified with $C_{1-5}$ alkyl or benzyl groups.

Preferred examples of the cycloalkylene groups represented by - Ⓑ - are five-membered and six-membered rings, which may be exemplified by cyclopentane-1, 3-ylene and cyclohexane-(1,3- or 1,4-)ylene preferably cyclohexane-1,4-ylene, while the cycloalkenylene groups as represented by - Ⓑ - preferably include five-membered and six-membered rings, which may be exemplified by cyclopenten-(1,3-, 1,4- or 3,5-)ylene, cyclohexen-(1,3-, 1,4-, 1,5-, 3,5- or 3,6-)ylene, cyclopentadien-(1,3, 1,4-or 2,5-)ylene, 1,3-cyclohexadien(1,3-, 1,4-, 1,5-,2,4-, 2,5- or 2,6-)ylene and 1,4-cyclohexadien-(1,3-, 1,4- or 1,5-)ylene preferably cyclopentadien-2,5-ylene or cyclohexadien-1,4-ylene.

The cycloalkylene and cycloalkenylene groups as represented by - Ⓑ - may have one to two substituents, being same or different, at their replacable positions. The substituted phenylene groups as represented by - Ⓑ - desirably have one to two substituents, being same or different. As the said substituent, there may be mentioned, for example, alkyl groups having a number of carbon atoms of 1 to 3 (e.g., methyl, ethyl, propyl and isopropyl groups), alkenyl groups having a number of carbon atoms of 2 to 3 (e.g., vinyl, 1-methylvinyl, 1-propenyl, allyl, and allenyl groups), alkynyl groups hving a number of carbon atoms of 2 to 3 (e.g., ethynyl, 1-propynyl and propargyl groups), cyclopropyl, halogens (e.g., chlorine, bromine, fluorine and iodine), methoxy, dimethylamino, trifluoromethyl, 2-ethoxyethyl and the like, preferably methyl halogens, metoxy or trifluoromethyl.

Preferably example of - Ⓑ - is halogenophenylene or cyclohexane.

Preferably in compounds as the general formula (1) hereinbefore compounds as the general formula:

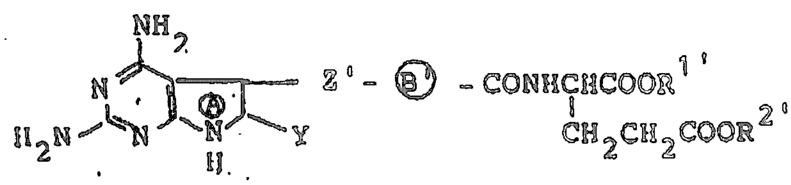

wherein $-COOR^{1'}$ or $-COOR^{2'}$ each is the same as or different from the other and represents a carboxyl group which may be esterified with a $C_{1-5}$ alkyl group or benzyl; - Ⓑ'- is a $C_5$ or $C_6$-cycloalkylene group or a halogeno phenylene ; Z' is a $C_{2-4}$ alkylene group, or its salt. In the above formula, as the carboxyl group represented by $-COOR^1$ and $-COOR^2$ which may be esterified with $C_{1-5}$ alkyl group or benzyl, there are mentioned, for example, carboxyl groups being esterified with methyl, ethyl, propyl, iso-propyl, n-butyl, iso-butyl, sec-butyl, tert-butyl, n-pentyl, iso-pentyl, sec-pentyl preferably $C_{1-3}$ alkyl group, for example methyl or ethyl and benzly:

The $C_5$ or $C_6$-cycloalkylene groups represented by - Ⓑ'- are a five-membered or six-memberd ring which may be examplified by cyclopentane-1,3-ylene and cyclohexane-(1,3-or 1,4-)ylene, preferably cyclohexane-1,4-ylene.

Referring to the above formulae, halogen of halogenophenylene of - Ⓑ'- is chlorine, bromine, fluorine and iodine, preferably chlorine.

Rferring to the above formulae, examples of the $C_{2-4}$ alkylene as represented by Z' are ethylene, trimethylene and tetramethlene, preferably trimethlene.

More specifical examples of compounds are shown in the Table 1 described below.

Table 1

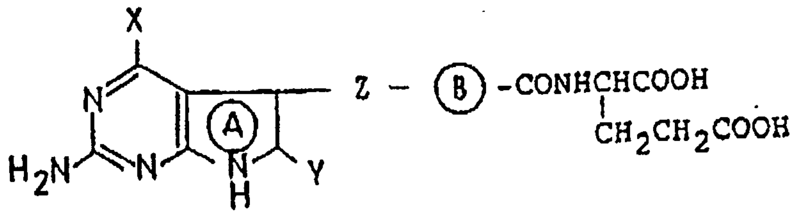

| No. | X | (A) | Z | (B) |
|---|---|---|---|---|
| 1 | $NH_2$ | pyrrole | $-(CH_2)_3-$ | 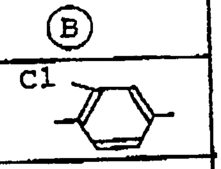 |
| 2 | $NH_2$ | pyrrole | $-(CH_2)_3-$ | 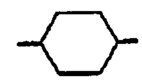 |
| 3 | $NH_2$ | pyrrole | $-CH_2CH_2CH-$<br>$CH_3$ | 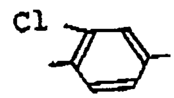 |
| 4 | $NH_2$ | pyrrole | $-CH_2CH_2CH-$<br>$CH_2CH_3$ | 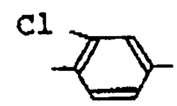 |
| 5 | $NH_2$ | pyrroline | $-(CH_2)_3-$ | 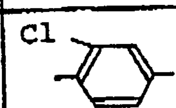 |
| 6 | OH | pyrrole | $-(CH_2)_3-$ | 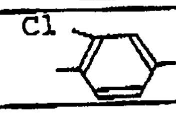 |
| 7 | OH | pyrrole | $-CH_2CH_2CH-$<br>$CH_3$ | 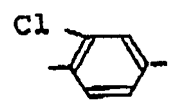 |
| 8 | OH | pyrroline | $-CH_2CH_2CH-$<br>$CH_3$ | 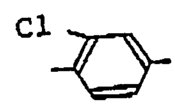 |
| 9 | $NH_2$ | pyrrole | $-(CH_2)_3-$ | 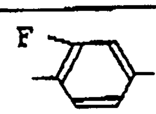 |
| 10 | $NH_2$ | pyrrole | $-CH_2CH_2CH-$<br>$CH_3$ | 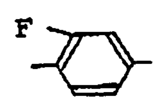 |
| 11 | $NH_2$ | pyrrole | $-CH_2CH_2CH-$<br>$CH_2CH_3$ | 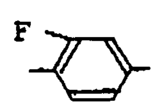 |

Table 1 (continued)

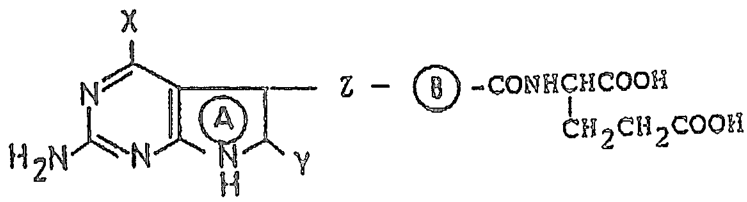

| No. | X | Ⓐ | Z | Ⓑ |
|---|---|---|---|---|
| 12 | $NH_2$ | pyrroline | $-(CH_2)_3-$ | 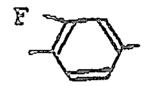 |
| 13 | OH | pyrrole | $-(CH_2)_3-$ | 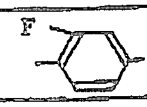 |
| 14 | OH | pyrrole | $-CH_2CH_2CH(CH_3)-$ | 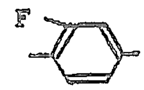 |
| 15 | OH | pyrroline | $-CH_2CH_2CH(CH_3)-$ | 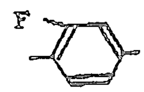 |
| 16 | $NH_2$ | pyrroline | $-(CH_2)_3-$ | 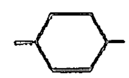 |
| 17 | OH | pyrrole | $-(CH_2)_3-$ | 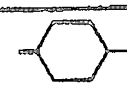 |
| 18 | $NH_2$ | pyrrole | $-(CH_2)_3-$ | 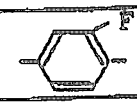 |
| 19 | $NH_2$ | pyrrole | $-CH_2CH_2CH(CH_3)-$ | 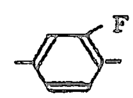 |
| 20 | $NH_2$ | pyrrole | $-CH_2CH_2CH(CH_2CH_3)-$ | 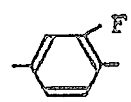 |
| 21 | $NH_2$ | pyrroline | $-(CH_2)_3-$ | 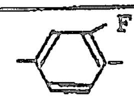 |
| 22 | OH | pyrrole | $-(CH_2)_3-$ | 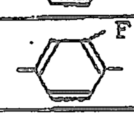 |
| 23 | OH | pyrrole | $-CH_2CH_2CH(CH_3)-$ | 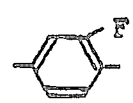 |

Table 1 (continued)

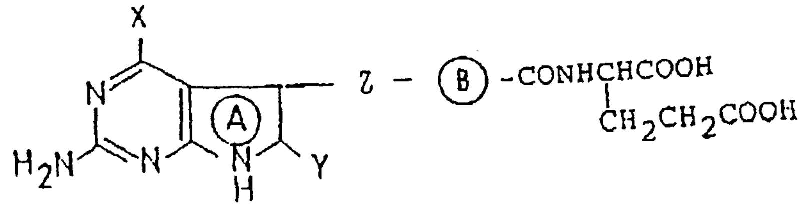

| No. | X | (A) | Z | (B) |
|---|---|---|---|---|
| 24 | OH | pyrroline | $-CH_2CH_2CH(CH_3)-$ | 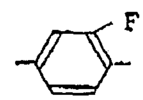 |
| 25 | $NH_2$ | pyrrole | $-(CH_2)_3-$ | 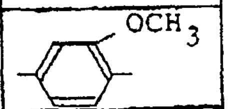 |
| 26 | $NH_2$ | pyrrole | $-CH_2CH_2CH(CH_3)-$ | 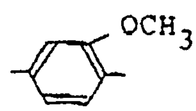 |
| 27 | $NH_2$ | pyrrole | $-CH_2CH_2CH(CH_2CH_3)-$ | 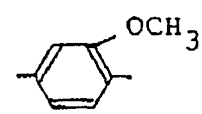 |
| 28 | $NH_2$ | pyrroline | $-(CH_2)_3-$ | 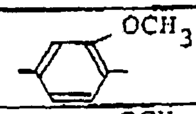 |
| 29 | OH | pyrrole | $-(CH_2)_3-$ | 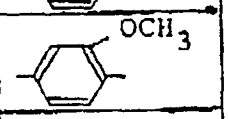 |
| 30 | OH | pyrrole | $-CH_2CH_2CH(CH_3)-$ | 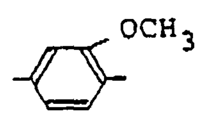 |
| 31 | OH | pyrroline | $-CH_2CH_2CH(CH_3)-$ | 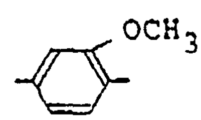 |

Described in the following are the procedures of producing the compounds (I) of the present invention or their salts.

The compounds (I) or their salts can be obtained by acylating a glutamic acid derivative represented by the formula (III) with a carboxylic acid represented by the formula (II) or its reactive derivative in respect to the carboxyl group. The above-mentioned acylating means include, for example, a procedure of acylating the compound (III) with the compound (II) or its reactive derivative in the presence of carbodiimides, diphenylphosphoryl acid azide or diethyl phosphorocyanidate. The used amount of the compound (III) is normally in the range of about 1 to 20 mole equivalents against the compound (II) or its reactive derivative, preferably about 1 to 5 mole equivalents. The carbodiimides may desirably be used generally in the proportions of about 1 to 25 mole equivalents against the compound (II), preferably about 1 to 25 mole equivalents.

As the said carbodiimides, dicyclohexylcarbodiimide is preferred from the standpoint of practical use,

and use may also be made of other carbodiimides, such as diphenylcarbodiimide, di-o-tolylcarbodiimide, di-p-tolylcarbodiimide, di-tert-butylcarbodiimide, 1-cyclohexyl-3-(2-morpholinoethyl)carbodiimide, 1-cyclohexyl-3-(4-diethylaminocyclohexyl)carbodiimide, 1-ethyl-3-(2-diethylaminopropyl)carbodiimide and 1-ethyl-3-(3-diethylamino-propyl)carbodiimide. The acylation reaction is preferably carried out in an appropriate solvent, and as the solvent, for example, use can be made of water, alcohols (e.g., methanol and ethanol), ethers (e.g., dimethyl ether, diethyl ether, tetrahdyrofuran, dioxane, monoglyme and diglyme), nitriles (e.g., acetonitrile), esters (e.g., ethyl acetate), halogenated hydrocarbons (e.g., dichloromethane, chloroform and carbon tetrachloride), aromatic hydrocarbons (e.g., benzene, toluene and xylene), acetone, nitromethane, pyridine, dimethylsulfoxide, dimethylformamide, hexamethylphosphoramide, sulfolane or suitable solvent mixtures thereof. This reaction can be normally conducted over a pH range of about 2 to 14, preferably 6 to 9, and at a reaction temperature ranging from about -10 °C to the boiling point (up to about 100 °C) of the solvent used, preferably about 0 to 50 °C, for a length of time of about 1 to 100 hours, preferably about 4 to 50 hours. The pH value of the reaciton solution is suitably adjusted, as the case may be, with use of acids (e.g., hydrochloric acid, sulfuric acid, phosphoric acid, nitric acid and acetic acid), bases (e,g., sodium methylate, sodium ethylate, sodium hydroxide, potassium hydroxide, barium hydroxide, lithium hydroxide, sodium carbonate, potassium carbonate, barium carbonate, calcium carbonate, sodium hydrogencarbonate, trimethylamine, triethylamine, triethanolamine and pyridine) or buffers (e.g., phosphate buffer, borate buffer and acetate buffer) and the like. The reaction can be allowed to proceed more advantageously by utilizing a catalyst capable of accelerating the acylation. As the said catalyst, for example, there may be mentioned base catalysts and acid catalysts. Examples of such base catalysts include tertiary amines (e.g., aliphatic tertiary amines, such as triethylamine; and aromatic tertiary amines, such as pyridine, $\alpha$-, $\beta$- or $\gamma$-picoline, 2,6-lutidine, dimethylaminopyridine, 4-(1-pyrrolidinyl)pyridine, dimethylaniline and diethylaniline), while those of the acid catalysts include Lewis acids (e.g., anhydrous zinc chloride, anhydrous aluminum chloride ($AlCl_3$), anhydrous ferric chloride, titanium tetrachloride ($TiCl_4$), tin tetrachloride ($SnCl_4$), antimony pentachloride, cobalt chloride, cupric chloride and boron trifluoride etherate) and the like. Among the above-described catalysts, 4-dimethylaminopyridine or 4-(1-pyrrolidinyl)pyridine in many cases is peferred. The used amount of the catalyst desirably is in the neighborhood of such a catalytic quantity as may be able to accelerate the acylation and normally is in the range of about 0.01 to 10 mole equivalents against the compound (II), preferably about 0.1 to 1 mole equivalent. As the reactive derivative of the carboxylic acid (II) in respect to the carboxyl group, there may be mentioned, for example, derivatives of the carboxylic acids (II), such as their acid halides (e.g., fluorides, chlorides, bromides and iodides), their acid anhydrides (e.g., anhydrous iodoacetic acid and anhydrous isobutyric acid), their acid anhydrides with lower-mono-alkyl carbonates (e.g., monomethylcarbonate, monoethylcarbonate, monopropylcarbonate, monoisopropylcarbonate, monobutyl- carbonates, monoiso-butylcarbonate, monosec-butylcarbonate and monotert-butylcarbonate), their active esters (e.g., cyanomethyl ester, ethoxycarbonylmethyl ester, methoxymethyl ester, phenyl ester, o-nitrophenyl ester, p-nitrophenyl ester, p-carbomethoxyphenyl ester, p-cyanophenyl ester and phenylthio ester), their acid azides, their acid anhydrides with phosphoric acid diesters (e.g., dimethyl phosphate, diethyl phosphate, dibenzyl phosphate and diphenyl phosphate), their acid anhydrides with phosphorous acid diesters (e.g., dimethyl phosphite, diethyl phosphite, dibenzyl phosphite and diphenyl phosphite), and others. In the acylating means utilizing such reactive derivatives, the solvent, pH, catalyst, reaction temperature, etc. are the same as those in the acylation in the presence of carbodiimides as described previously.

In the production of the compound (I) or its salt where $-COOR^1$ and $-COOR^2$ are a carboxyl group, or the compound (I-1) or its salt, it is preferable to react the compound (III) where $-COOR^1$ and $-COOR^2$ are an esterified carboxyl group with the compound (II) or its reactive derivative in respect to the carboxyl group, followed by per se known decomposition reaction or catalytic reduction reaction to conduct deesterification. Examples of the said decomposition reaction include hydrolysis reaction (Method A) under basic conditions, hydrolysis reaction (Method B-1) under acidic conditions and decomposition reaction (Method B-2) under acidic, nonaqueous conditions. Examples of the bases which are usable in the Method A include metal alkoxides, such as sodium methoxide, sodium ethoxide, sodium butoxide and potassium butoxide, and metal hydroxide, such as sodium hydroxide, potassium hydroxide, lithium hydroxide and barium hydroxide, and amines, such as ammonia, triethylamine and pyridine, and as the acids which are utilizable in the Method B-1, there may be mentioned, for example, mineral acids, such as hydrochloric acid, hydrobromic acid, sulfuric acid, nitric acid and phosphoric acid, and organic acids, such as trifluoroacetic acid, trichloroacetic acid, methanesulfonic acid, benzenesulfonic acid, p-toluenesulfonic acid and camphorsulfonic acid, while examples of the catalysts which are employable in the Method B-1 include mineral acids, such as hdyrochloric acid, hydrobromic acid, perchloric acid, sulfuric acid, nitric acid and phosphoric acid, organic acids, such as trifluoroacetic acid, trichloroacetic acid, methanesulfonic acid, benzenesulfonic acid,

p-toluenesulfonic acid and camphorsulfonic acid, and Lewis acids, such as anhydrous zinc chloride, anhydrous aluminum chloride ($AlCl_3$), anhydrous ferric chloride, titanium tetrachloride ($TiCl_4$), tin tetrachloride ($SnCl_4$), antimony pentachloride, cobalt chloride, cupric chloride and boron trifluoride etherate. Any decomposition reaction according to either of the above described methods is carried out by allowing the reaction to proceed in an appropriate solvent at a temperature in the range of 0° C to the boiling point of the solvent used, preferably 10 to 80° C, for 30 min to 2 days, preferably 1 to 24 hours. As the reaction solvent, used in the Methods A and B-1 are, for example, water, methanol, ethanol, propanol, butanol, ethylene glycol, methoxyethanol, ethoxyethanol, tetrahydrofuran, dioxane, monoglyme, diglyme, pyridine, diemthylformamide, dimethylsulfoxide, sulfolane and suitable solvent mixtures thereof, whereas in the case of the Method B-2, there are utilizable, for example, ethyl acetate, diemthyl ether, diethyl ether, tetrahydrofuran, dioxane, monoglyme, diglyme, dichloromethane, chloroform, carbon tetrachloride, acetonitrile, benzene, toluene, xylene, nitromethane, pyridine or suitable solvent mixtures thereof. The said catalytic reduction reaction, (Method C) is carried out in an appropriate solvent at a temperature in the range of about -40° C to the boiling point of the reaction solvent used, preferably about 0 to 50° C. Examples of the solvents which are usable in the reaction include water, alcohols (e.g., methanol, ethanol, propanol, iso-propanol, butyl alcohol, sec-butyl alcohol, tert-butyl alcohol, ethylene glycol, methoxyethanol and ethoxyethanol), acetates (e.g., methyl acetate and ethyl acetate), ethers (e.g., dimethyl ether, diethyl ether, tetrahydrofuran, dioxane, monoglyme and diglyme), aromatic hydrocarbons (e.g., benzene, toluene and xylene), pyridine, diemthylformamide and suitable solvent mixtures thereof. As the catalyst for the catalytic reduction, there are used, for example, palladium, platinum, rhodium and Raney nickel. On the occasion of the use of such catalysts, addition of minute amounts of acetic acid, trifluoroacetic acid, hydrochloric acid, sulfuric acid, etc., in some instances, can permit the reaction to proceed advantageously. It depends upon the properties of $-COOR^1$ and $-COOR^2$ which reaction method should be adopted to produce the compound (I-1). Normally, when $-COOR^1$ and $-COOR^2$ are a carboxyl group esterified with a methyl, ethyl, propyl, butyl, sec-butyl, phenyl or substituted phenyl group, the Method A or B-1 can be applied advantageously, and in cases where $-COOR^1$ and $-COOR^2$ are a carboxyl group esterified with an iso-propyl or tert- butyl group, the Method B-2 is advantageously applicable, while in the case of a carboxyl group esterified with a benzyl or substituted benzyl group, the Method B-1 or C can be employed advantageously. When $-COOR^1$ and $-COOR^2$ each is diifferent from the other, a suitable combination of the above described Methods A, B-1, B-2 and C is desirably used.

Below given is the explanation on the method of producing the starting compound (II).

The compound (II) can be produced for example by means of the raection steps shown in the following:

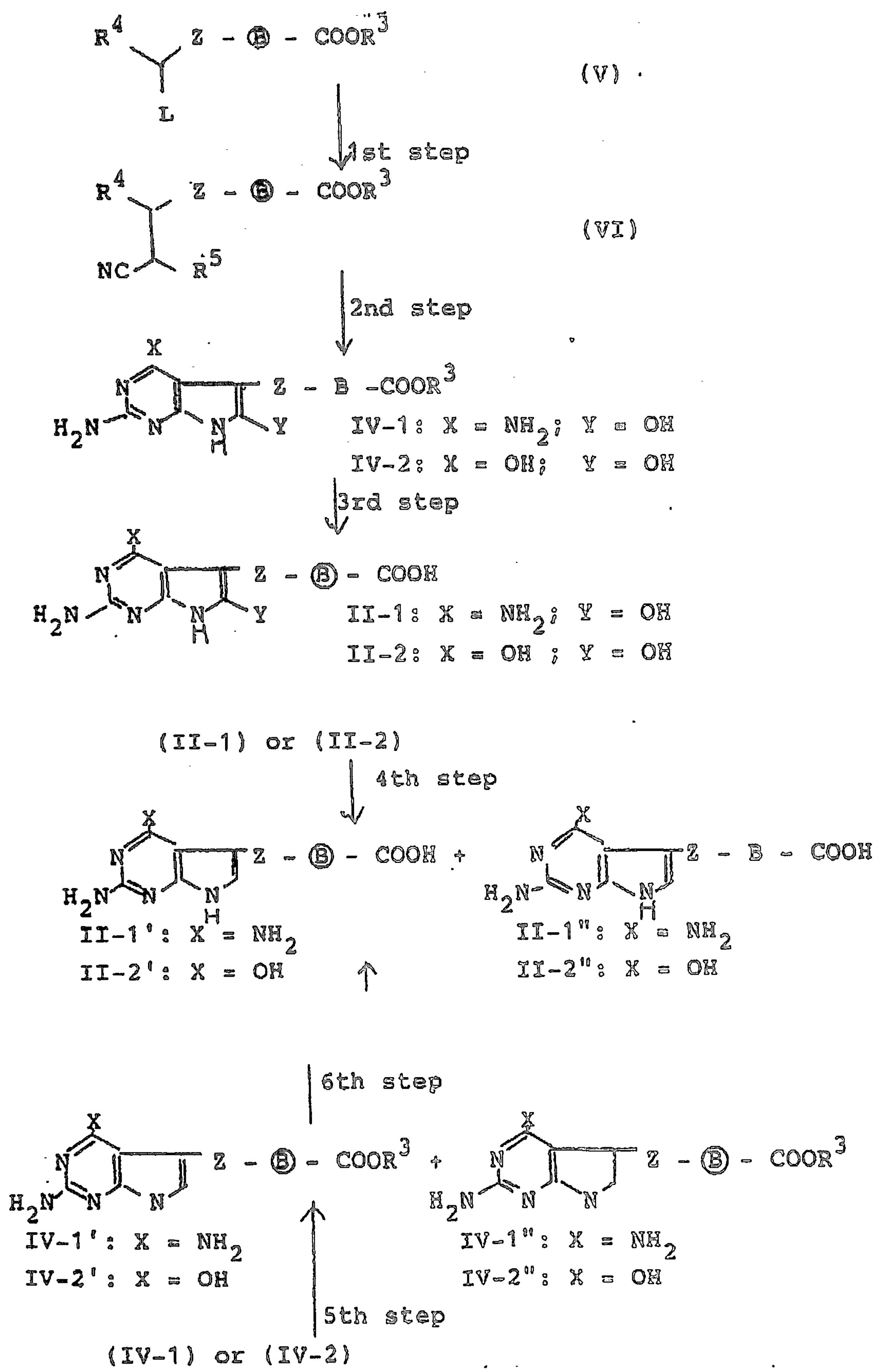


In the above reaction steps, X, Y, $R^3$, the ring - Ⓑ - and Z are as defined hereinbefore, and $R^4$ is an esterified carboxyl group represented by the formula $-COOR^6$, while $R^5$ is a cyano group or an esterified carboxyl group represented by the formula $-COOR^6$, with L being a halogen atom (e.g., chlorine, bromine

and iodine atoms) or a removabale group (e.g., methanesulfonyloxy, benzenesulfonyloxy, p-toluenesulfonyloxy and trifluoromethanesulfonyloxy groups) capable of being easily derived from the hydroxyl group. $R^6$ in the esterified carboxyl group as represented by the formula $-COOR^6$ includes, for example, lower alkyls of $C_2$ to $C_4$ ( e.g., methyl, ethyl, propyl, isopropyl, butyl, sec-butyl and tert-butyl), benzyl and substituted benzyls (e.g., p-nitrobenzyl and p-methoxybenzyl).

The above-mentioned reaction steps are described below in detail.

The 1st step:

The starting compound (V) can be subjected to a condensation reaction with malononitrile or cyanoacetate ($NC\text{-}CH_2COOR^6$ wherein $R^6$ is as defined hereinbefore) under basic conditions to convert into the compound (VI). With reference to the base, solvents reaction conditions, etc. to be employed, the per se known procedure can be adopted.

The 2nd step:

The compound (VI), upon treatment with guanidine, reacts with its cyano or ester group and then undergoes ring-closure and cyclization to form anew a pyrrolo[2,3-d]pyrimidine ring. On the occasion of ring-closure, basic reaction conditions can permit the reaction to proceed advantageously. Examples of the usable base include metal alkoxides, such as sodium methoxide, sodium ethoxide and potassium tert-butoxide. The reaction medium includes, for example, methanol, ethanol, propanol, tert-butylalchol, dimethyl-sulfoxide and hexamethylphosphoramide, and the reaction temperature ranges from 0 to 150 °C, preferably from 20 to 100 °C, while the reaction time is in the region of 1 to 48 hours, preferably 2 to 24 hours.

The 3rd step:

The compound (IV-1: X = $NH_2$, Y = OH, or IV-2: X = OH, Y = OH) as obtained in the 2nd step, after having its ester residue undergo a deesterification reaction as employed in the production of the compound (I-1), can be convereted to th compound (II-1: X = $NH_2$, Y = OH, or II-2: X = OH, Y = OH).

The 4th step:

The compound (II-1 or II-2) as obtained in the 3rd step can be subjected to a reduction reaction to produce the compound (II-1'and II-1": Y = $NH_2$, Y = H, or II-2' or II-2": X = OH, Y = H). Conditions of the reduction reaction are knwon per se, and the reduction reaction with use of metal hydrides (e.g., borane, alane or their ate complexes) and the like can be applied.

Meanwhile, the 3rd and 4th steps may be carried out backward with the order of application being reversed; in the 5th step, the compound (IV-1 or IV-2) is treated through a reduction reaction similar to the one in the 4th step to give the compound (IV-1' and IV-1": X = $NH_2$, Y = H, or IV-2' and IV-2": X = OH, Y = H), which, then in the 6th step, is subjected to the same deesterification reaction as conducted in the 3rd step to produce the compound (II-1' and II-1", or II-2' and II-2"). Depending upon the properties of substituents in the compound (IV-1 or IV-2), one can choose arbitrarily which should first be carried out, the deesterification reaction or reduction reaction.

Out of the above-mentioned compounds (II) and (IV), the compound where Y is hydrogen can also be produced by means of the reaction steps shown below.

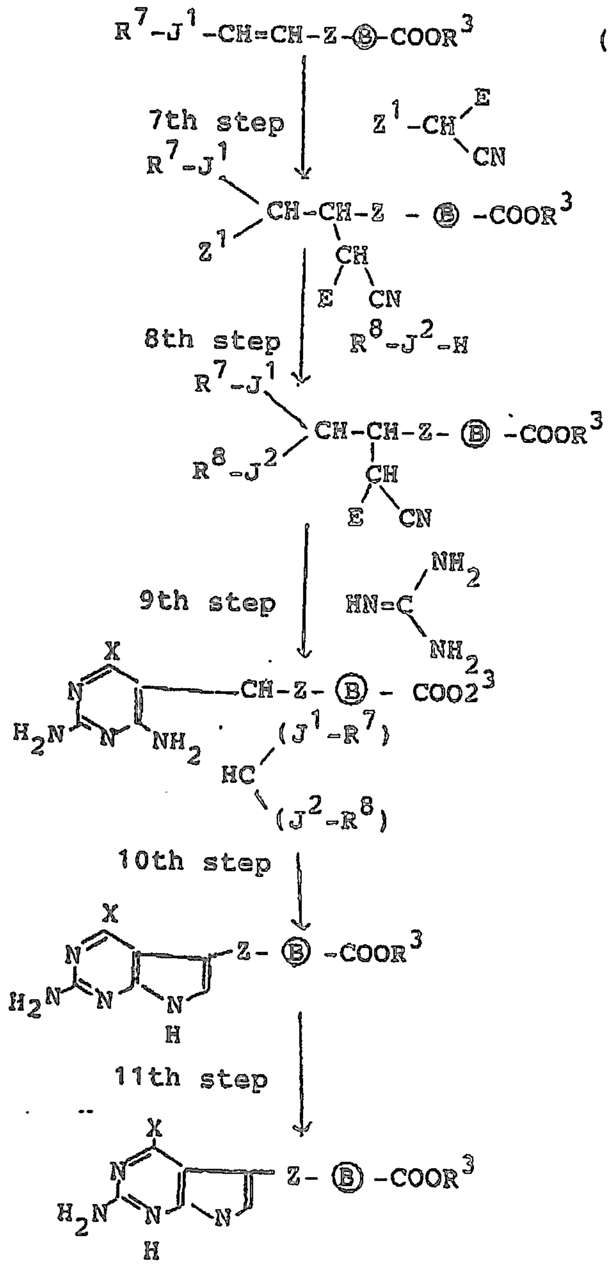


(VII)

(VIII)

(IX)

(X)

(IV)

(IV''')

In the above reaction steps, X, $R^3$, the ring - Ⓑ - and Z are as defined hereinbefore; $J^1$ and $J^2$ each is different from the other and represents oxygen or sulfur; $R^7$ and $R^8$ each is different from the other and representes a hydrocarbn residue; $Z^1$ is a halgeon atom (e.g., chlorine, bromine and iodine); and E is a cyano group or a group of the formulae $-COOR^9$, $-CSOR^9$ or $-CSSR^9$. As the hydrocarbon residue represented by $R^7$ and $R^8$, there may be mentioned lower alkyl groups having a number of carbon atoms of 1 to 5 (e.g., methyl, ethyl, propyl, isopropyl, n-butyl, iso-butyl, sec-butyl, tert-butyl, n-pentyl, iso-pentyl, sec-pentyl, neo-pentyl and tert-pentyl), and benzyl and phenyl groups and the like. These lower alkyl groups and benzyl and phenyl groups may have one to three substitutes being same or different. Examples of such substituents include halogen atoms (e.g., fluorine, chlorine, bromine and iodine), nitro group, cyano group, alkoxy groups having a number of carbon atoms of 1 to about 4 (e.g., methoxy, ethoxy, propoxy, iso-propoxy, n-butoxy, iso-butoxy, sec-butoxy and tert-butoxy groups), alkyl groups having a number of carbon atoms of 1 to about 4 (e.g., methyl, ethyl, propyl, isopropyl, butyl, isobutyl, sec-butyl and tert-butyl groups), alkanoyl groups having a number of carbon atoms of 1 to about 4 (e.g., formyl, acetyl, propionyl, n-butyryl and iso-butyryl groups) and trifluoromethyl group. $R^9$ in the formulae $-COOR^9$, $-CSOR^9$ and $-CSSR^9$ includes hydrocarbon residues being described in detail as $R^7$ and $R^8$.

The above reaction steps are described below in detail.

The 7th step:

This step involves a step where

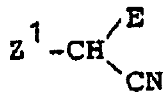

is allowed to add to the double bond of the compound (VII) ($R^7$-$J^1$-CH = CH-) to produce the compound (VII). The used amount of

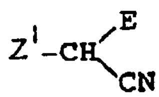

against the compound (VII) normally is in the range of about 0.5 to 4 mole equivalents, preferably about 0.8 to 1.5 mole equivalents. This reaction can be carried out by allowing the reaction to proceed in an appropriate solvent at a reaction temperature in the range of about -10° C to the boiling point of the used solvent, prefereably about 0° C to 50° C, for about 30 min to 48 hours, preferably 1 to 24 hours. As the solvent for the reaction, there may be used, for example, alcohols (e.g., methanol and ethanol), ethers (e.g., dimethyl ether, diethyl ether, tetrahydrofuran, dioxane, monoglyme and diglyme), nitriles (e.g., acetonitrile), esters (e.g., ethyl acetate), halogenated hydrocarbons (e.g., dichloromethane, chloroform and carbon tetrachloride), aromatic hydrocarbons (benzene, toluene and xylene) or suitable solvent mixtures thereof. In carrying out the reaction, light radiation or addition of organic peroxides can also permit the reaction to proceed more advantageously. The said organic peroxides include, for example, t-butyl hypochloride, peracetic acid, perbenzoic acid and p-chloroperbenzoic acid. The compound (VII) obtained by the above procedure is relatively reactive, and can be used in the following step directly without being isolated, although it may be isolated.

8th step:

The compound (VII) as obtained in the 7th step can be reacted with alcohols or thiols as represented by $R^8$-$J^2$-H in an appropriate solvent at a reaction temperature in the range of about -10° C to the boiling point (up to about 100° C) of the used solvent, preferably about 0 to 50° C, for about 10 min to 24 hours, preferably 20 min 12 hours, to produce the compound (IX). As the solvent which is usable in the reaction, there are used, for example, ethers (e.g., dimethyl ether, diethyl ether, tetrahydrofuran, dioxane, monoglyme and diglyme), nitriles (e.g., actonitrile), esters (e.g., ethyl acetate), halogenated hydrocarbons (e.g., dichloromethane, chloroform and carbon tetrachloride)aromatic hydrocarbons (e.g., benzene, toluene and xylene) or suitable solvent mixtures thereof. Alternately, the alcohols or thiols themselves, as representedby $R^8$-$J^2$-H, may be used in excess as a solvent.

9th step:

The compound (IX), upon treatment with guanidine in a suitable solvent, reacts with the cyano group, ester residue or thioester residue and undergoes cyclization, simultaneously with the formation of a pyrimidine ring, to thereby produce the compound (X). In conducting this reaction, the reaction temperature is held at 0 to 150° C, preferably 20 to 100° C, while the reaction time is in the range of 1 to 48 hours, preferably 2 to 24 hours. Under basic conditions, the reaction can be allowed to proceed advantageously. Examples of the base, which is usable, include metal alkoxides, such as sodium methoxide, sodium ethoxide and potassium tert-butoxide. As the reaction solvent, there may be used, for example, methanol, ethanol, propanol, tert-butylalcohol dimethylsulfoxide, hexamethylphosphoramide or their suitable sovlent mixtures.

10th step:

The 10 step constitutes the reaction which comprises restoring a group of

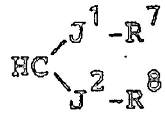

in the compound (X) to a carbonyl group (>C=O) and also cusing spontaneously a intramolecular ring-closure reaction to thus convert the compound (X) to the compound (IV). The said restoration reaction to a carbonyl group can be carried out, for example, by subjecting the compound (X), either in itself or in an appropriate solvent, to a decomposition reaction at a reaction temperature in the range of about -10°C to the boiling point (up to about 100°C) of the used reaction solvent, preferably about 0 to 50°C, for a length of time in the region of about 10 min to 100 hours, preferably 20 min 48 hours. The said decomposition reaction includes, for example, hydrolysis reaction under acidic conditions (Method B-1), decomposition reaction under acidic, nonaqueous conditions (Method B-2), catalytic reduction reaction (Method C), decomposition reaction (Method D) with use of metal salts or decomposition reaction (Method E) with use of oxidizing agents. The Methods B-1, B-2 and C can be conducted into practice by following the procedures, as described in detail in the decomposition reactions for $-COOR^1$ and $-COOR^2$, as such. Examples of the metal salts, which are utilizable in the Method D, include cupric chloride, silver nitrate, silver oxide, mercuric chloride and tellurium salts (e.g., tellurium nitrate and tellurium trifluoroacetate), while as the oxidizing agents being usable in the Method E, there may be mentioned, for example, oxygen-light, hydrogen peroxide, perbenzoic acid, m-chloroperbenzoic acid, perchlorates (e.g., lithium perchlorate, mercuric perchlorate and tetrabutyl perchlorate ammonium), nitrosylsulfuric acid, alkyl nitrites (e.g., isoamyl nitrite), halogen (e.g. iodine, bromine, chlorine), N-bromo-succinimide, sulfuryl chloride and chloramine T. Depending upon the chemical properties of $-J^1-R^7$ and $-J^2-R^8$, one can choose arbitrarily which method should be adopted to restore to a carbonyl group (>C=O). As the reaction solvents being usable in the Methods D and E, there may be used, for example, water, alcohols (e.g., methanol, ethanol, propanol, iso-propanol, butanol, sec-butanol, tert-butanol, ethylene glycol, methoxyethanol and ethoxyethanol), ethers (e.g., dimethyl ether, diethyl ether, tetrahydrofuran, dioxane, monoglyme and diglyme), aromatic hydrocarbons (e.g., benzene, toluene and xylene), halogenated hydrocarbons (e.g., dichloromethane, chloroform and carbon tetrachloride), acetone, acetonitrile or suitable sovlent mixtures thereof. The intramolecular ring-closure reaction in the production step of the compound (IV), which takes place in the course of or after restoration to the carbonyl group (<C=O), normally involves spotaneous condensation with the amino group in the pyrimidine ring to form a pyrrolo[2,3-d]-pyrimidine ring. On the occasion of this, the presence of an acid catalyst can permit the ring-closure reaction to proceed rapidly and in improved yields. The acid catalyst includes, for example, mineral acids, organic acids or Lewis acids as described in detail for the Methods B-1 and B-2.

11th step:

The compound (IV) containing a pyrrole ring as the ring Ⓐ as obtained in the 10th step can be subjected, if necessary, to a catalytic reduction reaction to convert easily to the compound (IV") having a pyrroline ring as the ring Ⓐ. In cases where - Ⓑ - is a cycloalkenylene group or a phenylene group having a substituent, such groups can be subjected to a catalytic reduction reaction in this step or either of the steps from the 1st through the 10th steps to thereby convert to the corresponding cycloalkylene or cycloalkenylene group. When - Ⓑ - is a non-substituted cyclohexanylene or non-substituted cyclohexenylene group, such groups can also be obtained by subjecting a non-substituted phenylene group to a catalytic reduction reaction. As the said catalytic reduction reaction, the previously mentioned Method C as such can be applied advantageously.

Also, the compound (IV) or compound (IV") can be subjected to a deesterification reaction in the same manner as described in the 3rd step to convert to the compound (II) or compound (II"), if necessary.

The reactions, reagents and reaction conditions, which are conducted into practice or employed in the 1st step through 10th step or in the production steps of the starting compounds, (III), (V) and (VI), as well as the protective groups for individual functional groups which are applied, as the occasion needs, are already known and described in great detail in the following literature: J. F. W. McOmine, "Protective Groups in Organic Chemistrty", published by Plenum Press, London and New York (1973); Pine, Hendrickson and Hammond, "Organic Chemistry" 4th edition, vol. I and II, published by Hirokawa Shoten of Japan (1982); and M. Fieser and L. Fieser, "Reagents for Organic Synthesis", vol. 1 to 13, published by Wiley Interscience of New York, London, Sydney and Toronto (1969 - 1988).

Furthermore, the amino, hydroxyl and mercapto groups represented by X in the compounds (I), (II) and

(IV) can be converted to one another, as the case may be, by means of the substituent replacement reactions on the pyrimidine ring known in literature (suplement volume "Tanpakushitu/ Kakusan/Kouso - (Protein/Nucleic Acid/Enzymes), "ChemicalSynthesis of Nucleic Acids", published by Kyoritsu Publishing Co. of Japan (1968).

The compounds (I), (II) and (IV) of this invention as produced in these steps and the starting compounds used in each of the steps as well as products can be isolated from reaction mixtures by conventional separation and purification means, such as concentration, solvent extraction, chromatography and recrystallization.

The compounds (I), (II) and (IV) as obtained according to the produciton process fo this invention may form salts. Their salts with bases include, for example, salts formed with alkali metals, alkaline earth metals, non-toxic metals, ammonium and substituted ammoniums, such as sodium, potassium, lithium, calcium, magnesium, aluminum, zinc, ammonium, trimethylammonium, triethylammonium, triethanolammonium, pyridinium and pyridinium, while their satls with acids include, for example, salts formed with mineral acids, such as hydrochloric acid, sulfuric acid, nitric acid, phosphoric acid and boric acid, and organic acids, such as oxalic acid, tartaric acid, acetic acid, trifluoroacetic acid, methanesulfonic acid, benzenesulfonic acid, p-toluenesulfonic acid and camphorsulfonic acid.

The compounds (I) of this ivnention and their salts exhibit inhibitory activity against not less than one kind of enzymes utilizing folic acid and its related compounds as a substrate. Consequently, these compounds can be used either alone or in combination with other anti-tumor agents for the prupose of treatment of choriocarcinoma, leukemia, breast adenocarcinoma, head and neck cancer (epithelioma), squamocarcinoma, cellule lung cancer and lymphosarcoma as well as other various tumors.

The compounds (I) or their salts, when intended to be used as an anti-tumor agent, antimsteosarcoma, can be administered orally or parenterally, as such or after being processed into such dosage forms as powder, granule, tablet, capsule,suppository and injectable solution by measn of the conventional procedures with use of pharmacologically allowable carriers, excipients, diluents, etc. Their dosage amount varies depending upon the species of subject animals for example animal (e.g., mouse, rate, rabbit, dog, cat, human), type of diseases, symptoms, kind of compounds, route of administration, etc., and their daily dose for the above warm-blooded animals is about 2.0 to 100 mg/kg body weight, preferably 4.0 to 80 mg/Kg body weight, as the compound of this invention in the case of oral administration, while being about about 1.0 to 50 mg/kg body weight, preferably 20 to 40 mg/Kg body weight, in the case of parenteral administration. The method fo administration for injectable solution includes, for example, intrmuscular injection, intraperitoneal injection, subcutaneouis injection and intravenous injection.

The above-mentioned processing into pharmaceutical preparations is carried out in accordance with the per se known methods. In the manufacture of the above oral preparations, for example, tablets, binders (e.g., hydroxypropylcellulose, hydroxypropyl methylcellulose and macrogol), disintegrating agents (e.g., starch and carboxymethylcellulose calcium), lubricants (e.g., magnesium stearate adn talc) and the others can suitably be formulated.

In producing such non-oral, parenteral preparations as injectable solution, tonicity agents (e.g., glucose, D-sorbitol, D-mannitol and sodium chloride), preservatives (e.g., benzyl alcohol, chlorobutanol, methyl p-oxybenzoate and propyl p-oxybenzoate), buffering agents (e.g., phosphate buffer and sodium acetate buffer), etc. can suitrably be incorporated.

With reference to a specific example of the manufacture of tablets, for example, about 1.0 to 25 mg of the compound of this invention, 100 to 500 mg of lactose, about 50 to 100 mg of corn starch and about 5 to 20 mg of hydroxypropylcellulose, being weighed out for use in the manufacture of one tablet, are mixed, granulated and admixed with corn starch and magnesium stearate, followed by compression into a tablet weighing about 100 to 500 mg and measuring about 3 to 10 mm in diameter, in accordance with the conventional procedure. The resulting tablet can furthermore be processed into a entgeric-coated tablet by providing coating with use of a about 5 to 10 % concentrated solution of hydroxypropylmethylcellulose phthalate (about 10 to 20 mg per tablet) and castor oil (about 0.5 to 2 mg per tablet) in acetone-ethanol mixture.

Referring to a specific example of the preparation of injectable solution, for example, about 2.0 to 50 mg of sodium salt of the compound of this invention, as weighed out for use in the preparation of one ampoule, is dissolved in about 2 ml of isotonic saline, and the solution is filled into an ampoule, followed by fusion and heat sterilization at about 100 °C for about 30 min, or a solution of about 10 to 40 mg of mannitol or sorbitol in about 2 ml of sterilized distilled water is filled into an ampoule, followed by lyophilization and fusion. On the occasion of the use of the lyophilisated compound, the said ampoule is opened, and for example, the compound can be dissolved in isotonic saline to a concentration of about 1.0 to 25 mg/ml to prepare injectable solution intended for subcutaneous, intravenous or intramuscular administration.

[Reference Examples and Examples]

The present invention will be further explained by the following Reference Examples and Examples, but those Examples are mere examples and not to restrict the present invention in any manner, including variations to such extent as not deviating the scope of this invention.

In those Examples NMR spectrum was determined by Gemini 200 ( 200 MHz )-type spectrometer using tetramethylsilane as internal standard, and all the chemical shifts, δ values, were expressed in ppm downfield from internal tetramethyl silane.

Symbols in the Reference Example and Examples have respectively the meanings as follows:

| | |
|---|---|
| s | : singlet |
| d | : doublet |
| t | : triplet |
| q | : quartet |
| ABq | : AB type quartet |
| dd | : double doublet |
| dt | : double triplet |
| td | : triple doublet |
| ddd | : double double doublet |
| tdd | : triple double doublet |
| m | : multiplet |
| br. | : broad |
| brs | : broad singlet |
| J | : coupling constant |

Reference Example 1

Production of t-butyl 3-chloro-4-(5-methoxy-4-pentenyl)benzoate:

A 1.0 mole tetrahydrofuran solution (11.0 ml) of potassium tert-butoxide was added to a toluene solution (12 ml) of (methoxymethyl)triphenylphosphonium chloride (3.77 g) at 0° C, and after stirring was continued for 10 min, a toluene solution (10 ml) of t-butyl 3-chloro-4-(4-oxobutyl)benzoate (2.84 g) was added dropwise to the solution, folowed by stirring at 0° C for 20 min. The reaction solution was admixed with ether (40 ml), and the organic layer was separated, washed successively with water and saturated aqueosu solution of sodium chloride and then dried over anhydrous sodium sulfate. The solvent was distilled off under reduced pressure, and the resultant residue was treated with added hexane, whereupon the resulting triphenylphosphine oxide was filtered out. The filtrate was concentrated under reduced pressure, and the residue was purified by column chromatography (80 g of silica gel; ether:hexane = 1:20) to give the subject compound (1.92 g).

IR (Neat) : 2980, 2940, 2860, 1710, 1665, 1605, 860, 845 $cm^{-1}$.

$^1$H-NMR ($CDCl_3$) δ : 1.53-1.74(2H,m), 1.60(9H,s), 1.96(0.6H,dt,J = 7Hz,7Hz), 2.11-(0.4H,tdd,J = 7Hz,7Hz,7Hz), 2.65(2H,t,J = 8Hz), 3.50(1.8H,s), 3.58(1.2H,s), 4.35(0.4H, td,J = 7Hz,6Hz), 4.74-(0.6H,dt,J = 13Hz,7Hz), 5.90(0.4H,dt,J = 6Hz, 1Hz), 6.30(0.6H,d,J = 13Hz), 7.35(1H,d,J = 8Hz), 7.90(1H,d,J = 8Hz), 7.85(2H,s).

Reference Example 2

Production of t-butyl 3-chloro-4-[5,5-dicyano-4-(dimethoxymethyl)pentyl]benzoate:

Bromomalononitrile (1:27 g) and the compound (1.91 g) of Reference Example 1 were dissolved in dichloromethane (66 ml) under an atmosphere of argon, and after addition of molecualr sieve (3A, 1.0 g), the solution was irradiated with ultraviolet rays for 2 hours by use of a UV lamp for analytical use having a filter removed. Methanol (4 ml) was added to the reaction solution, which was stirred for 10 min and poured into ice water containing 2N aqueous potassium carbonate solution (5 ml), followed by extraction with dichloromethane. The organic layer was separated, washed with water and dried over anhydrous sodium sulfate, and the solvent was distilled off under reduced pressure. The resulting residue was purified by column chromatography (75 g of silica gel. ethyl acetate:hexane = 1:10) to produce the subject compound (2.08 g) in the form of a colorless oily substance.

IR (neat) : 2970, 2930, 2245, 1710, 1605, 860, 845 $cm^{-1}$.

$^1$H-NMR ($CDCl_3$)δ : 1.59(9H,s), 1.60-1.91(4H,m), 2.19-2.31(1H,m), 2.71(2H,t,J = 7Hz), 3.40(3H,s), 3.45-(3H,s), 4.10(1H,d,J = 4Hz), 4.30(1H,d,J = 5Hz), 7.36(1H,d,J = 8Hz), 7.90(1H,d,J = 8Hz), 7.96(1H,s).

Reference Example 3

Production of t-butyl 3-chloro-4-[4-(2,4,6-triaminopyrimidine-5-yl)-5,5-dimethoxypentyl]benzoate:

A tetrahydrofuran solution (6.70 ml) of 1.0 mole of potassium tert-butoxide was added to a tert-butyl alcohol suspension (30 ml) of guanidine hydrochloride (640 mg) under an atmosphere of argon, and after stirring for 10 min, a tert-butyl alcohol solution (10 ml) of the compound (2.00 g) of Reference Example 2 was added to the solution, followed by heating under reflux for 2 hours. The reaction solution was added to water (200 ml) containing 1.0N aqueous potassium hydrogen sulfate solution (1 ml) and extracted with dichloromethane, and the organic layer was dried over anhydrous sodium sulfate. The solvent was distilled off under reduced pressure, and the resulting residue was purified by column chromatography (50 g of silica gel. dichloro- methane:methanol =30:1 - 15:1) to give the subject compound (2.27 g) in the form of colorless amorphous powder.

IR (KBr) : 3480, 3375, 3150, 2980, 2940, 1715, 1605, 1570, 1435, 850, 805 $cm^{-1}$.

$^1$H-NMR ($CDCl_3$) $\delta$ : 1.40-1.65(3H,m), 1.59(9H,m), 1.73-2.06(1H,m), 2.60(2H,t,J=7Hz), 2.79-(1H,ddd,J=11Hz,3Hz,1Hz), 3.45(3H,s), 3.50(3H,s), 4.33(1H,J=3Hz), 4.45(4H,brs), 5.17 (2H,brs), 7.34-(1H,d,J=8Hz), 7.88(1H,d,J=8hz), 7.94(1H,s).

Example 1

Production of diethyl N-[3-chloro-4-[3-(2,4-diamino-7H-pyrrolo[2,3-d]pyrimidine-5-yl)propyl]benzoyl]-L-glutamate:

The compound (200 mg) of Reference Example 3 was dissolved in trifluoroacetic acid (1 ml) and water (20 mg), and the solution was stirred at room temperature for 2 hours. The trifluoroacetic acid was distilled off under reduced pressure, followed by drying under vacuum, and the resulting residue and diethyl glutamate hydrochloride (172 mg) were suspended in diemthylformamide (2 ml). A dimethylformamide solution (2 ml) of diethyl phosphorocyanidate (82 mg) was added to the suspension at 0°c, followed by stirring for 15 min. Then, a dimethylformaide solution (2 ml) of triethylamine (218 mg) was added dropwise to the mixture at the same temperature, followed by stirring at 0°C for 30 min and at room temperature for 2 hours, and the solvent was distilled off under reduced pressure. The resulting residue was purified by column chromatography (15 g silica gel. dichloromethane separated from conc. aqueous ammonia dichloromethane:ethanol containing 10 % ammonia = 40:1 - 30:1) to give the subject compound (175 mg) in the form of colorless amorphous powder.

IR (KBr) : 3320, 3160, 1735, 1630, 1575, 1540, 1500, 1200 $cm^{-1}$.

$^1$H-NMR ($CDCl_3$) $\delta$ : 1.17(3H,t,J=7Hz), 1.21(3H,t,J=7Hz), 1.82-2.20(4H,m), 2.42(2H,t,J=7Hz), 2.66-(2H,t,J=7Hz), 2.72(2H,t,J=7Hz), 4.67(2H,g,J=7Hz), 4.12(2H,g,J=7Hz), 4.34-4.51(1H,m), 5.33(2H,s), 5.95-(2H,s), 6.40(1H,s), 7.38(1H,d,J=8Hz), 7.82(1H,d,J=8Hz), 7.88(1H,s), 8.62(1H,d,J=8Hz), 10.49(1H,s).

Example 2

Production of N-[3-chloro-4-[3-(2,4-diamino-7H-pyrrolo[2,3-d]pyrimidin-5-yl)propyl]benzoyl]-L-glutamic acid:

1.0N aqueous sodium hydroxide solution (0.497 ml) was added to a tetrahydrofuran-water mixed solution (2:1, 3 ml) of the compound (80 mg) of Example 1, followed by stirring at room temperature for 1 hour. The solution mixture was concentrated to the total volume of 1.0 ml under reduced pressure, and after the resulting insoluble matter was filtered out through a Millipore filter, the filtrate was cooled to 0°C, followed by addition of acetic acid (0.1 ml). The resulting crystals were recovered by filtration, washed adequately with water and dried under reduced pressure to give the subject compound (64 mg) in the form of wahite crystals.

IR (KBr) : 3340, 3200, 2940, 1660, 1632, 1540, 1500, 1397 $cm^{-1}$.

$^1$H-NMR ($Me_2SO-d_6$) $\delta$ : 1.76-2.21(4H,m), 2.34(2H,t,J=7HZ) 2.68(2H,t,J=7Hz), 2.72(2H,t,J=7Hz), 4.29-4.48(1H,m), 5.50(2H,brs), 6.13(2H,s), 6.45(1H,s), 7.41(1H,d,J=8Hz), 7.84(1H,d,J=8Hz), 7.90(1H,s), 8.50-(1H,d,J=8Hz), 10.53(1H,s).

Example 3

Production of diethyl N-[4-[3-(2,4-diamino-7H-pyrrole[2,3-d]pyrimidin-5-yl)propyl]cyclohexylcarbonyl]-L-gultamate:

The compound (531 mg) of Example 1 was dissolved in acetic acid and and after addition of platinum

dioxide (100 mg), the solution was stirred severely at 40 °C for 15 hours under hydrogen atmosphere. After the catalyst was removed and the solvent was distilled off under reduced pressure, the resultant residue was purified by columm chromatography (53 g of silica gel ; chroloform : ethanol = 19:1) to give the subject compound (180 mg).

1 R (KBr) 3330, 3165, 1730, 1630, 1570, 1540 $cm^{-1}$

$^1H$-NMR ($Me_2SO$-$d_6$)δ : 1.15(3H,t,J=7Hz), 1.19(3H,t,J=7Hz), 1.26-2.15(16H,m), 2.26-2.39(2H,m), 2.54-2.67(2H,m), 4.03(2H,q,J=7Hz), 4.10(2H,q,J=7Hz), 4.14-4.27(1H,m), 5.35(2H,s), 5.90(2H,s), 6.38-(1H,d,J=1.8Hz), 7.96-8.09(1H,m), 10.34(1H,d,J=8Hz)

Example 4

Production of N-[4-[3(2,4-diamino-7H-pyrrolo[2,3-d]pyrimidin-5-yl)propyl]cyclohexylcarbonyl]-L-gultamic acid:

Using the compound of Example 3 (151 mg), was conducted the similar reaction as Example 2 to give the subject compound (112 mg).

1 R (Kbr) 3340, 3250, 2930, 1655, 1630, 1540 $cm^{-1}$

$^1H$-NMR ($Me_2SO$-$d_6$) δ : 1.30-2.12(16H,m), 2.27(2H,t,J=7Hz), 2.52-2.67(2H,m), 4.10-4.23(1H-m), 5.54-(2H,s), 6.14(2H,s), 6.41(1H,s), 7.80-7.93(1H,m), 10.47(1H,s)

## Claims

1. A compound represented by the formula:

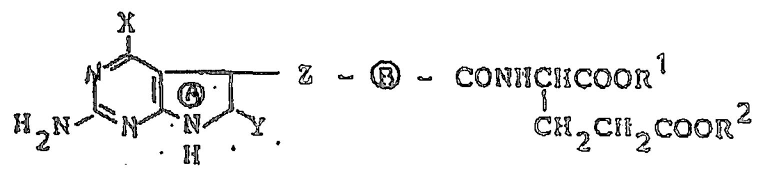

wherein the ring Ⓐ is a pyrrole ring which may be hydrogenated; X is an amino, hydroxyl or mercapto group; Y is a hydrogen atom or a hydroxyl group; -$COOR^1$ and -$COOR^2$ each is the same as or different from the other and represents a carboxyl group which may be esterified; - Ⓑ - is a cycloalkylene group which may be substituted, a cycloalkenylene group which may substituted or a phenylene group which is substituted; Z is a divalent $C_{2-4}$ aliphatic group of straight chain which may be substituted, or its salt.

2. A compound or its salt according to Claim 1, wherein $R^1$ or $R^2$ is a $C_{1-5}$ alkyl, benzyl or phenyl which may be substituted with a nitro or methoxy group; - Ⓑ - is a substituted or unsubstituted 5- or 6-membered cycloalkylene or cycloalkenylene group, or a substituted phenyl group, the substituent(s) being one or two of $C_{1-3}$ alkyl, $C_{2-3}$ alkenyl or $C_{2-3}$ alkynyl, cyclopropyl, halogen, methoxy, dimethylamino, trifluoromethyl, oxo, formyl, methoxymethyl and ethoxyethyl; and Z is a divalent $C_{2-4}$ aliphatic group of straight chain which may be substituted with one or two of $C_{1-3}$ alkyl, $C_{2-3}$ alkenyl or $C_{2-3}$ alkynyl, cyclopropyl, fluorine, hydroxy, oxo, methoxy, dimethylamino, diethylamino, trifluoromethyl, formyl, hydroxymethyl, 2-hydroxyethyl, methoxymethyl and 2-ethoxyethyl.,

3. A process for producing a compound of Claim 1, which comprises reacting a compound of the formula,

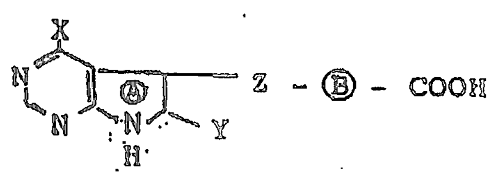

wherein the rings Ⓐ is a pyrrole ring which may be substituted; X is an amino, hydroxyl or mercapto group; Y is a hydrogen atom or a hydroxyl group; - Ⓑ - is a cycloalkylene group which may be substitueted, a cycloalkenylene group which may be substituted or a phenylene group which is substituted; Z is a divalent $C_{2-4}$ aliphatic group of straight chain which may be substituted, or its

reactive derivative in respect to the carboxyl group with a compound of the formula:

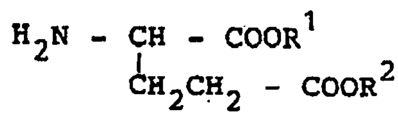

wherein -$COOR^1$ and -$COOR^2$ each is the same as or different from the other and represents a carboxyl group which may be esterified.

**4.** An anti-tumor agent comprising a compound according to Claim 1 or its pharmaceutically acceptable salt and a pharmaceutically acceptable diluent or carrier.

**5.** A compound as represented by the general formula:

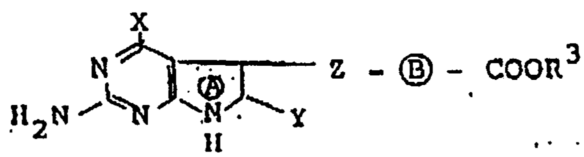

wherein the ring Ⓐ is a pyrrole ring which may be hydrogenated; X is an amino, hydroxyl or mercapto group; Y is a hydrogen atom or a hydroxyl group; - Ⓑ - is a cycloalkylene group which may be substituted, a cycloalkenylene group which maybe substituted or a phenylene group which is substituted; $COOR^3$ is a carboxyl which may be substituted; Z is a divalent $C_{2-4}$ aliphatic group of straight chain which may be substituted] or its salt.

**6.** A compound according to Claim 5, wherein $R^3$ is a $C_{1-5}$ alkyl, benzyl or phenyl which may be substituted with a nitro or methoxy group; - Ⓑ - is a substituted or unsubstituted 5- or 6-membered cycloalkylene or cycloalkenylene group which may be substituted or phenyl group which is substituted with one or two of $C_{1-3}$ alkyl, $C_{2-3}$ alkenyl or alkynyl, cyclopropyl, halogen, methoxy, dimethylamino, trifluoromethyl, oxo, formyl, methoxymethyl and ethoxyethyl; and Z is a divalent $C_{2-4}$ aliphatic group of straight chain which may be substituted with one or two of $C_{1-3}$ alkyl $C_{2-3}$ alkenyl or alkynyl, cyclopropyl, fluorine, hydroxy, oxo, methoxy, dimethylamino, diethylamino, trifluoromethyl, formyl, hydroxymethyl, 2-hydroxyethyl, methoxymethyl, 2-ethoxyethyl.

**7.** A compound according to Claim 1, wherein the ring Ⓐ is a pyrroloe ring.

**8.** A compound according to Claim 1, wherein the ring Ⓐ is a pyrroline ring.

**9.** A compound according to Claim 1, wherein X is an amino group.

**10.** A compound according to Claim 1, wherein X is a hydroxyl group.

**11.** A compound according to Claim 1, wherein X is a mercapto group.

**12.** A compound according to Claim 1, wherein Y is a hydrogen atom.

**13.** A compound according to Claim 1, wherein Y is a hydroxyl group.

**14.** A compound according to Claim 1, wherein -$COOR^1$ and -$COOR^2$ are carboxyl groups.

**15.** A compound according to Claim 1, wherein -$COOR^1$ and -$COOR^2$ are esterified carboxyl groups.

**16.** A compound according to Claim 1, wherein - Ⓑ - is a cycloalkylene group which may be substituted.

**17.** A compound according to Claim 1, wherein. - Ⓑ - is a cycloalkenylene group which may be substituted.

**18.** A compound according to Claim 1, wherein - Ⓑ - is a phenylene group which is substituted.

**19.** A compound according to Claim 12, wherein X is an amino or hydroxyl group; Y is a hydrogen atom; - Ⓑ - is a cyclohexenylene, methoxyphenylene or halogenophenylene group; Z is $C_{2-4}$ alkylene which may be substituted with $C_{1-3}$ alkyl, or its salt.

**20.** A compound according to Claim 1, wherein X is an amino group; Y is a hydrogen atom; $-COOR^1$ or $-COOR^2$ each is the same or different from the other and represents a carboxyl group which may be esterified with a $C_{1-5}$ alkyl group or benzyl; - Ⓑ - is a cyclohexenylene or halogenohenylene group; Z is a $C_{2-4}$ alkylene group, or its salt.

**21.** Diethyl N-[3-chloro-4-[3(2,4-diamino-7H-pyrrolo[2,3-d]pyridine-5-yl)-propyl]benzoyl]-L-glutamate or its salt.

**22.** N-[3-Chloro-4-[3(2,4-diamino-7H-pyrrolo-[2,3-d]pyridine-5-yl)propyl]benzoyl]-L-glutamic acid

**23.** Diethyl N-[4-[3-(2,4-diamino-7H-pyrrolo[2,3-d]pyrimidine-5-yl)propyl]cyclohexylcarbonyl]-L-glutamate

**24.** N-[4-[3-(2,4-Diamino-7H-pyrrolo[2,3-d]-pyrimidine-5-yl)propyl]cyclocarbonyl]-L-glutamic acid

**25.** A process for producing a compound according to Claim 5, which comprises (1) reacting a compound represented by the formula:

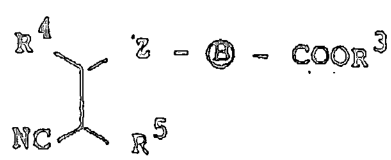

wherein $R^3$ is a carboxyl group which may be esterified; $R^4$ is a carboxyl group which may be esterified; $R^4$ is a cyano group or a carboxyl group which may be esterified; - Ⓑ - is a cycloalkylene group which may be substituted, a cycloalkenylene group which may be substituted or phenylene group which is substituted; Z is a divalent $C_{2-4}$ aliphatic group of straight chain which may be substituted, or a salt thereof with guanidine, or (2) allowing a compound represented by the formula:

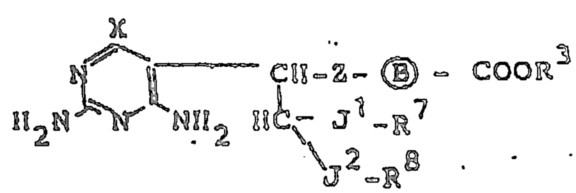

wherein $R^3$ , - Ⓑ - and Z are the same as defined above; X is an amino, hydroxyl or mercapto group; $R^7$ and $R^8$ each is the same or different from the other and represents a hydrocarbon group; $J^1$ and $J^2$ each is the same or different from the other and represents oxygen or sulfur atom, or a salt thereof to undergo a ring-closure reaction and if necessary, followed by a deesterification reaction or/and a reduction reaction in an optional order.

**26.** Use of a compound of Claim 1 or its pharmaceutically acceptable salt for the preparation of antitumor agent.